(19) **Europäisches Patentamt / European Patent Office / Office européen des brevets**

(11) **EP 1 688 089 B1**

(12) **EUROPEAN PATENT SPECIFICATION**

(45) Date of publication and mention
of the grant of the patent:
**17.09.2008  Bulletin 2008/38**

(51) Int Cl.:
***A61B 5/022*** *(2006.01)*

(21) Application number: **06001870.2**

(22) Date of filing: **30.01.2006**

(54) **Cuff for blood pressure monitor, manufacturing method thereof and blood pressure monitor**

Manschette für Blutdruckmessgerät, Herstellungsverfahren hierfür und Blutdruckmessgerät

Brassard de sphygmomanomètre, procédé de fabrication du dernier et sphygmomanomètre

(84) Designated Contracting States:
**DE ES FR GB IT**

(30) Priority: **04.02.2005  JP 2005029500**

(43) Date of publication of application:
**09.08.2006  Bulletin 2006/32**

(73) Proprietor: **Omron Healthcare Co., Ltd.**
**Kyoto 615-0084 (JP)**

(72) Inventors:
• **Karo, Hiromichi**
c/oOmron Healthcare Co.,Ltd.
**Kyoto 615-0084 (JP)**
• **Sano, Yoshihiko**
c/oOmron Healthcare Co.,Ltd.
**Kyoto 615-0084 (JP)**
• **Kishimoto, Hiroshi**
c/oOmron Healthcare Co.,Ltd.
**Kyoto 615-0084 (JP)**
• **Tsurumi, Yoshinori**
c/oOmron Healthcare Co.,Ltd.
**Kyoto 615-0084 (JP)**

(74) Representative: **Kilian, Helmut**
**Wilhelms, Kilian & Partner**
**Patentanwälte**
**Eduard-Schmid-Strasse 2**
**81541 München (DE)**

(56) References cited:
• **PATENT ABSTRACTS OF JAPAN vol. 2000, no. 25, 12 April 2001 (2001-04-12) & JP 2001 231757 A (OMRON CORP), 28 August 2001 (2001-08-28)**
• **PATENT ABSTRACTS OF JAPAN vol. 1997, no. 09, 30 September 1997 (1997-09-30) & JP 09 117419 A (MATSUSHITA ELECTRIC WORKS LTD), 6 May 1997 (1997-05-06)**
• **PATENT ABSTRACTS OF JAPAN vol. 014, no. 310 (C-0736), 4 July 1990 (1990-07-04) & JP 02 107226 A (MATSUSHITA ELECTRIC WORKS LTD), 19 April 1990 (1990-04-19)**

EP 1 688 089 B1

**Description**

BACKGROUND OF THE INVENTION

Field of the Invention

The present invention relates to a cuff for a blood pressure monitor wound around a measurement site of a living body such as a wrist or an upper arm at the time of measurement of blood pressure, and a manufacturing method of the cuff. The present invention also relates to a blood pressure monitor having the cuff.

Description of the Background Art

Normally, to measure a blood pressure value, a cuff provided with a fluid bag for pressing an artery located within a living body is wound around the body surface, and arterial pressure pulse waves caused in the artery by inflation/deflation of the fluid bag are detected to measure the blood pressure value. Here, the cuff refers to a band-shaped structure having a bladder, which can be wound around a part of a living body, for use in measurement of arterial pressure of an upper limb, a lower limb or the like by introducing fluid such as gas or liquid into the bladder. Thus, the cuff represents the concept including the fluid bag as well as members for winding the fluid bag around the living body. Particularly, the cuff wound around and fitted on a wrist or an upper arm is also called an arm band or a manchette.

The cuff for a blood pressure monitor has a bag-shaped cover member containing an air bag as the fluid bag therein. The bag-shaped cover member is usually formed into a bag shape by stacking two sheet-shaped members constituting an inner cover and an outer cover one on the other and connecting their rims. The cuff for a blood pressure monitor having such a configuration is disclosed, e.g., in Japanese Patent Laying-Open No. 09-117419.

In recent years, it is common to provide the cuff for a blood pressure monitor with a curled elastic member, identified as an elastic member, within the bag-shaped cover member and on the outer side of the air bag, so as to facilitate the fitting operation of the cuff by the subject and also to allow the air bag to expand smoothly toward the living body at the time of pressurizing the air bag after fitting of the cuff (see, e.g., Japanese Patent Laying-Open No. 2002-209858). As the curled elastic member, a plate-shaped member made of resin, which is wound annularly and changeable in size in a radial direction, may be used.

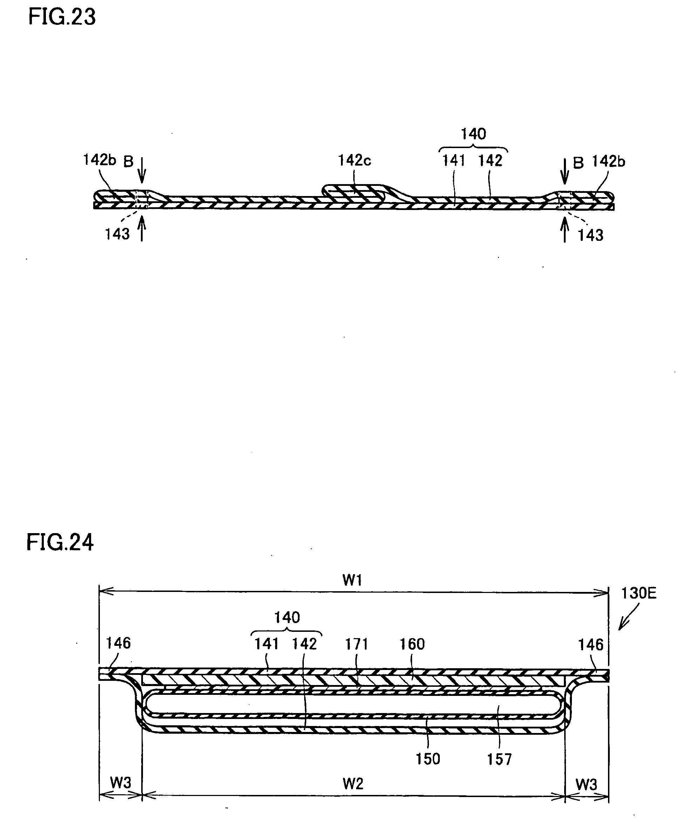

Figs. 24-26 each show a cross section of a cuff for a blood pressure monitor in the width direction when it is assumed that the above-described curled elastic member is provided inside the cuff disclosed in the above-described Japanese Patent Laying-Open No. 09-117419. Hereinafter, the configurations of the cuffs for a blood pressure monitor shown in Figs. 24-26 will be explained in detail as Conventional Examples 1-3, respectively.

In the cuff 130E for a blood pressure monitor according to Conventional Example 1 shown in Fig. 24, a bag-shaped cover member 140 contains therein a curled elastic member 160, and an air bag 150 that is attached and fixed to the inner peripheral surface of curled elastic member 160 via a double-faced tape 171. Air bag 150 has an inflated/deflated space 157 therein, which is inflated and deflated as the air flows in and out. Bag-shaped cover member 140 has two sheet-shaped members, which constitute an inner cover 142 forming an inner wall portion that is located on the living body side when cuff 130E is mounted to the living body, and an outer cover 141 forming an outer wall portion that is located outer than the inner wall portion. Bag-shaped cover member 140 is formed into a bag shape by stacking the sheet-shaped members constituting inner and outer covers 141 and 142 one on the other and connecting their rims by melting and bonding with high frequency wave or/and sewing and the like. At each end portion in the width direction of each of inner cover 142 and outer cover 141, connection allowance is provided where inner and outer covers 142 and 141 are connected together.

In the case of cuff 130E for a blood pressure monitor shown in Fig. 24 configured as described above, a connected portion 146 of inner and outer covers 142 and 141 protrudes outward at each end in the width direction of bag-shaped cover member 140. Thus, a width dimension W1 of cuff 130E for a blood pressure monitor is greater than a size W2 in the width direction of air bag 150 by two folds of a size W3 of the protruding portion at each end in the width direction of inner and outer covers 142 and 141 including the connection allowance. As a result, when the subject has clothes on, the hem of the clothes may touch the end portion of cuff 130E for a blood pressure monitor, making fitting of cuff 130E troublesome.

If cuff 130E for a blood pressure monitor is reduced in size in the width direction for the purpose of solving the problem, although the fitting property of cuff 130E may be improved, size W2 in the width direction of air bag 150 with respect to width dimension W1 of cuff 130E for a blood pressure monitor will become smaller correspondingly, since the connection allowance of the same size W3 needs to be maintained. This makes it difficult to secure a sufficient size in the width direction of air bag 150, causing degradation in performance of avascularization. As such, the above-described structure cannot necessarily be said to be a structure suitable for downsizing of the cuff for a blood pressure monitor in its width direction.

As described above, with the configuration shown in Fig. 24, the presence of the connection allowance hinders downsizing in the width direction of the cuff for a blood pressure monitor and, hence, production of a high-performance cuff for a blood pressure monitor excellent in fitting property. Thus, in Japanese Patent Laying-Open No. 09-117419 described above, the structures of the cufffor a blood pressure monitor as shown in Figs. 25 and 26 are proposed to solve the above-described problem.

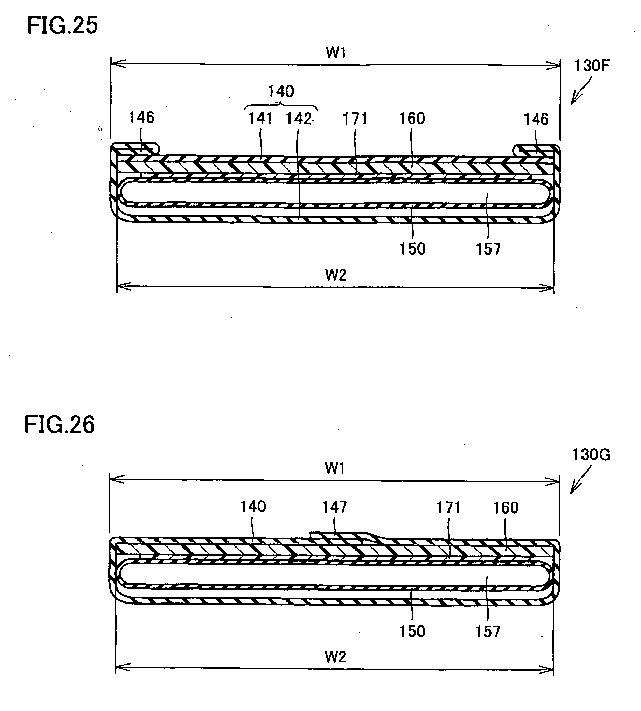

In a cuff 130F for a blood pressure monitor according to Conventional Example 2 shown in Fig. 25, inner cover 142 is made greater in width than outer cover 141, and the end portions of these two sheet-shaped members are connected together to form a bag-shaped cover member 140. Bag-shaped cover member 140 is then turned inside out, so that the connected portion 146 of inner and outer covers 142 and 141 is arranged on an inner side than the end in the width direction of bag-shaped cover member 140. With this configuration, connected portion 146 is located inner than each end portion in the width direction of air bag 150, and thus, it is possible to make size W2 in the width direction of air bag 150 sufficiently large with respect to width dimension W1 of cuff 130F for a blood pressure monitor. Accordingly, size W2 in the width direction of air bag 150 can be increased to the size substantially the same as width dimension W1 of cuff 130F for a blood pressure monitor.

In a cuff 130G for a blood pressure monitor according to Conventional Example 3 shown in Fig. 26, bag-shaped cover member 140 is formed of a single sheet-shaped member, of which one and the other ends in the width direction are connected together, and the resulting connected portion 147 is arranged on an inner side than the end in the width direction of bag-shaped cover member 140. With this configuration, connected portion 147 is located inner than each end portion in the width direction of air bag 150, and thus, size W2 in the width direction of air bag 150 can be made sufficiently large with respect to width dimension W1 of cuff 130G for a blood pressure monitor. As a result, it is possible to increase size W2 in the width direction of air bag 150 to the size substantially the same as width dimension W1 of cuff 130G for a blood pressure monitor.

When the configuration as shown in Fig. 25 is employed, however, bag-shaped cover member 140 needs to be turned inside out after connecting the sheet-shaped members together, which will complicate the manufacturing process. In particular, bag-shaped cover member 140 is usually made of cloth or the like, of which handling is not easy, making it extremely difficult to turn it inside out.

Further, when one of the configurations as shown in Figs. 25 and 26 is employed, there will be an unlevel portion on the outer peripheral surface of bag-shaped cover member 140. The unlevel portion would not only deteriorate handling property, as the finger may be caught when fitting the cuff, for example, but also cause curling or fraying of bag-shaped cover member 140. The unlevel portion may further adversely affect the appearance of the finished product.

In order to prevent occurrence of the unlevel portion on the outer peripheral surface of bag-shaped cover member 140, it may be conceivable to additionally provide a cover made of another member on the outer peripheral surface of bag-shaped cover member 140 to hide the same. Such a configuration however will not only increase the parts count but also complicate the assembly process, and will eventually press the manufacturing cost considerably.

A cuff according to the preamble of claim 1 is known from JP 2001 231 757 A and JP 09 117 419 A.

SUMMARY OF THE INVENTION

An object of the present invention is to provide a cuff for a blood pressure monitor that is easy to manufacture, suitable for reduction of cuff width, excellent in handling property at the time of fitting, and suppressing curling and fraying of a bag-shaped cover member, and a manufacturing method thereof, and to thereby provide at a low cost a blood pressure monitor of high performance and excellent in handling property and of which bag-shaped cover member is unlikely to be damaged.

Another object of the present invention is to provide a manufacturing method of a cuff for a blood pressure monitor, in the case where slack is formed in advance in a bag-shaped cover member to facilitate insertion of a fluid bag in a subsequent process step, which enables the slack to be formed easily.

A cuff for a blood pressure monitor according to the present invention is as defined in claim 1.

With this configuration, the fluid bag arranged inside the bag-shaped cover member can be made to have a size in the width direction approximately equal to the width dimension of the cuff for a blood pressure monitor. This ensures accurate measurement of the blood pressure values even if the cuff width is narrowed. It is thus possible to provide a cuff for a blood pressure monitor having a structure suitable for downsizing of the cuff, allowing improvement in handling property at the time of fitting. The cuff for a blood pressure monitor can be manufactured readily and inexpensively without the need of turning the bag-shaped cover member inside out that would otherwise complicate the manufacturing process. Further, the unlevel portion is not formed on the outer peripheral surface of the bag-shaped cover member, and accordingly, it is possible to provide a cuff for a blood pressure monitor of which bag-shaped cover member is unlikely to suffer curling or fraying, and thus having good appearance as a finished product.

In the cuff for a blood pressure monitor according to the present invention, the inner cover is preferably more elastic than the outer cover.

With this configuration, the inner cover expands to make it possible to readily insert the fluid bag and the elastic member into the bag-shaped cover member, even if the fluid bag and the elastic member each have the size substantially equal to the width dimension of the cuff for a blood pressure monitor. As such, it is possible to readily manufacture the cuff for a blood pressure monitor configured as described above.

In the cuff for a blood pressure monitor according to the present invention, in the deflated state where the fluid bag is not pressurized, the fluid bag preferably has a size in the width direction substantially equal to a width dimension of the cufffor a blood pressure monitor.

With this configuration, it is possible to provide a cuff for a blood pressure monitor having a structure suitable for reduction in width of the cuff, since the blood pressure values can be measured with accuracy even if the cuff width is decreased.

A blood pressure monitor according to the present invention includes: any of the cuffs for a blood pressure monitor described above; an inflating/deflating portion for inflating and deflating the fluid bag; a pressure detecting portion for detecting a pressure within the fluid bag; and a blood pressure value calculating portion for calculating a blood pressure value based on pressure information detected by the pressure detecting portion.

With this configuration, it is possible to inexpensively manufacture a highly reliable, high-performance blood pressure monitor that is excellent in handling property at the time of fitting.

A manufacturing method of a cuff for a blood pressure monitor according to a first aspect of the present invention is a manufacturing method of any of the cuffs for a blood pressure monitor described above. The method is as defined in claim 7.

When the cuff for a blood pressure monitor is manufactured using this manufacturing method, the slack is readily formed at the inner cover. Accordingly, it is possible to manufacture a cuff for a blood pressure monitor having a folded portion of the inner cover located between an end portion in the width direction of the outer cover and an end portion in the width direction of the elastic member, inexpensively and with good workability.

A manufacturing method of a cuff for a blood pressure monitor according to a second aspect of the present invention is a manufacturing method of any of the cuffs for a blood pressure monitor described above. The method is as defined in claim 8.

When the cuff for a blood pressure monitor is manufactured using this manufacturing method, the slack is readily formed at the inner cover. Accordingly, it is possible to manufacture a cuff for a blood pressure monitor having a folded portion of the inner cover located between an end portion in the width direction of the outer cover and an end portion in the width direction of the elastic member, inexpensively and with good workability.

According to the present invention, it is possible to provide a cuff for a blood pressure monitor that is easy to manufacture, suitable for reduction of cuff width, excellent in handling property upon fitting, and suppressing curing or fraying in its bag-shaped cover member. Accordingly, it is possible to inexpensively manufacture a high-performance blood pressure monitor that is excellent in handling property and of which bag-shaped cover member is unlikely to be damaged.

Further, according to the present invention, in the case where slack is formed in advance at the bag-shaped cover member to facilitate insertion of the fluid bag in a subsequent process step, the slack can be formed with ease.

The foregoing and other objects, features, aspects and advantages of the present invention will become more apparent from the following detailed description of the present invention when taken in conjunction with the accompanying drawings.

BRIEF DESCRIPTION OF THE DRAWINGS

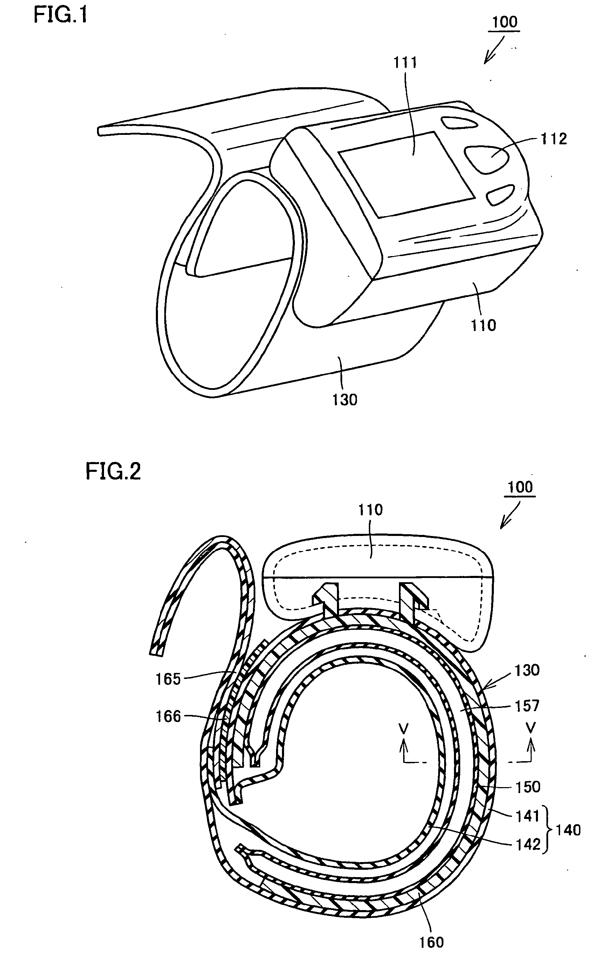

Fig. 1 is a perspective view showing a blood pressure monitor according to an embodiment of the present invention.
Fig. 2 is a vertical cross sectional view showing an inner structure of a cuff for a blood pressure monitor according to the embodiment of the present invention.

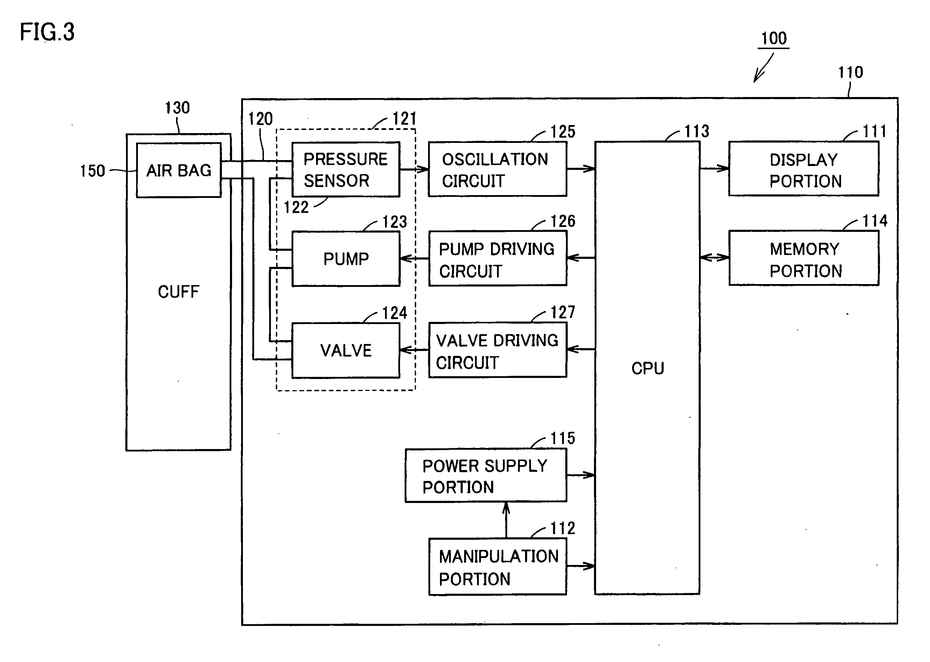

Fig. 3 is a block diagram showing a configuration of the blood pressure monitor according to the embodiment of the present invention.

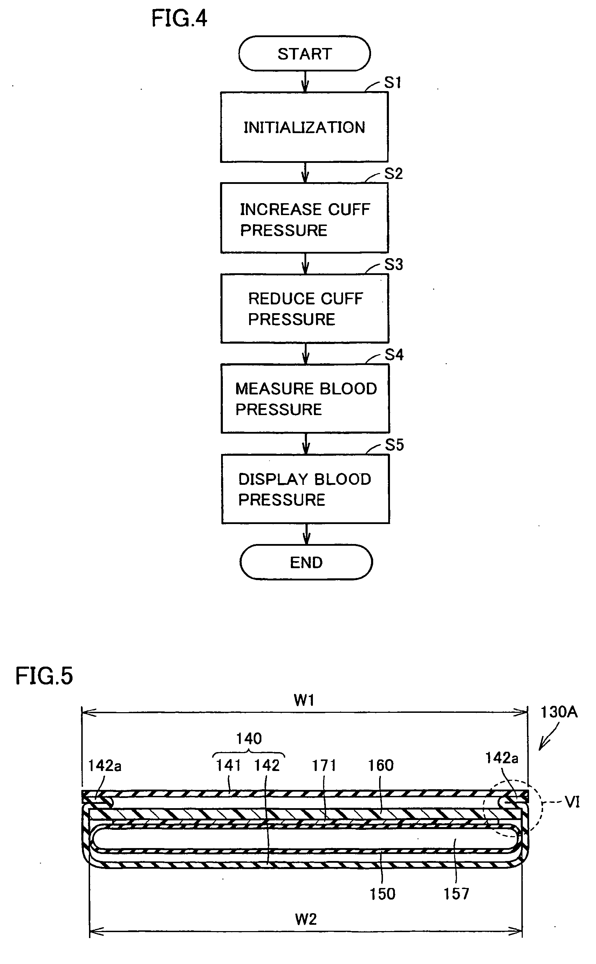

Fig. 4 is a flowchart illustrating a process flow of blood pressure measurement with the blood pressure monitor according to the embodiment of the present invention.
Fig. 5 is a schematic cross sectional view of a cuff for a blood pressure monitor according to Example 1 based on the embodiment of the present invention, taken along the line V-V in Fig. 2.

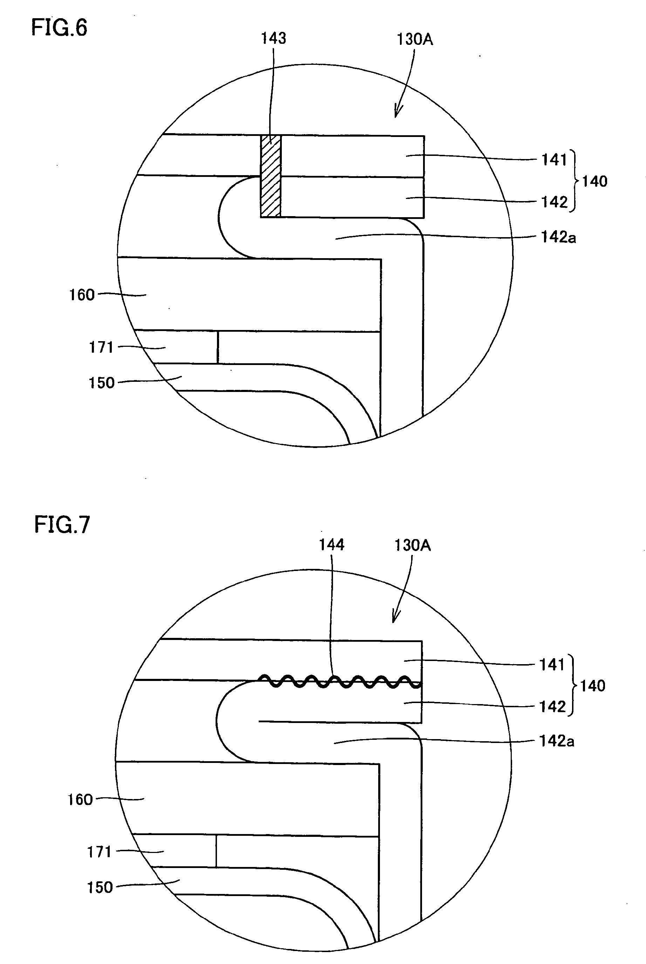

Fig. 6 is an enlarged cross sectional view of a region VI shown in Fig. 5, illustrating a configuration of a connected portion in the cuff for a blood pressure monitor according to Example 1 based on the embodiment of the present invention.
Fig. 7 is a schematic cross sectional view showing another configuration of the connected portion in the cuff for a blood pressure monitor according to Example 1 based on the embodiment of the present invention.

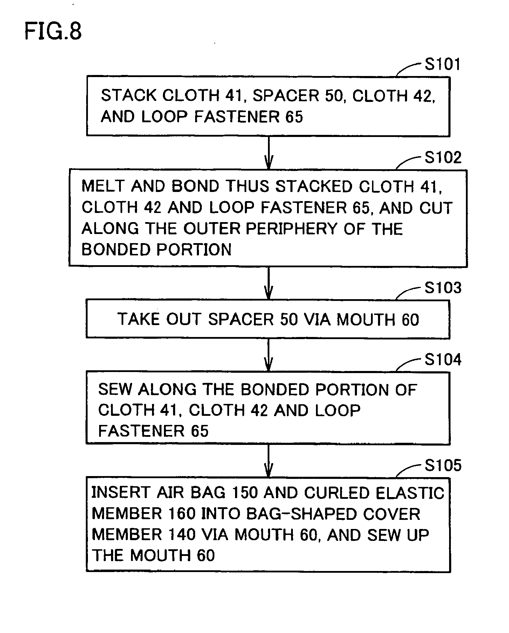

Fig. 8 is a flowchart illustrating a manufacturing method of the cuff for a blood pressure monitor according to Example 1 based on the embodiment of the present invention.

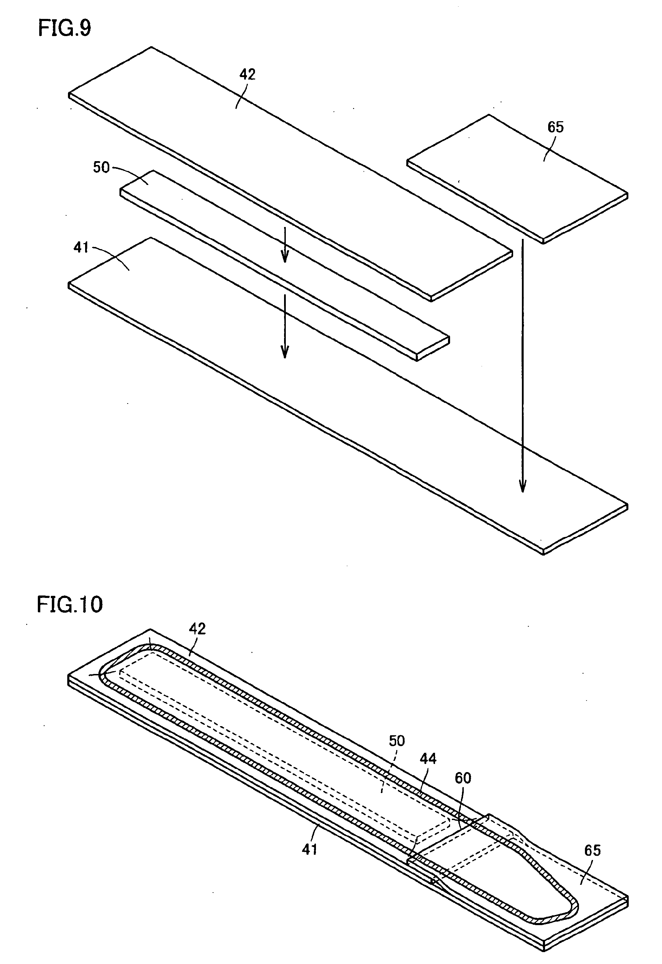

Fig. 9 is an exploded perspective view illustrating a process step in the manufacturing method of the cuff for a blood pressure monitor according to Example 1 based on the embodiment of the present invention.

Fig. 10 is a schematic perspective view illustrating a process step in the manufacturing method of the cuff for a blood pressure monitor according to Example 1 based on the embodiment of the present invention.

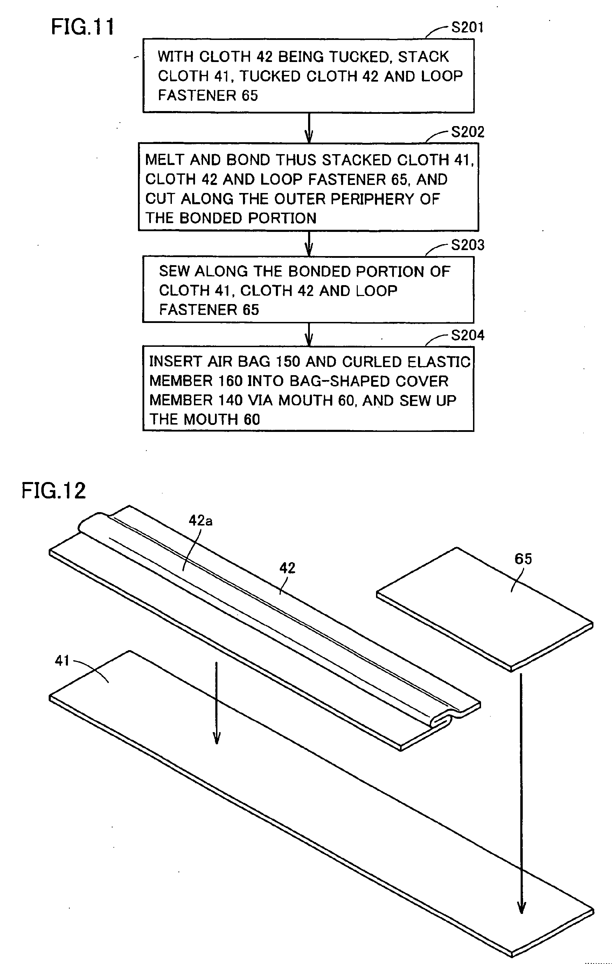

Fig. 11 is a flowchart illustrating another manufacturing method of the cuff for a blood pressure monitor according to Example 1 based on the embodiment of the present invention.

Fig. 12 is an exploded perspective view illustrating a process step in the other manufacturing method of the cuff for a blood pressure monitor according to Example 1 based on the embodiment of the present invention.

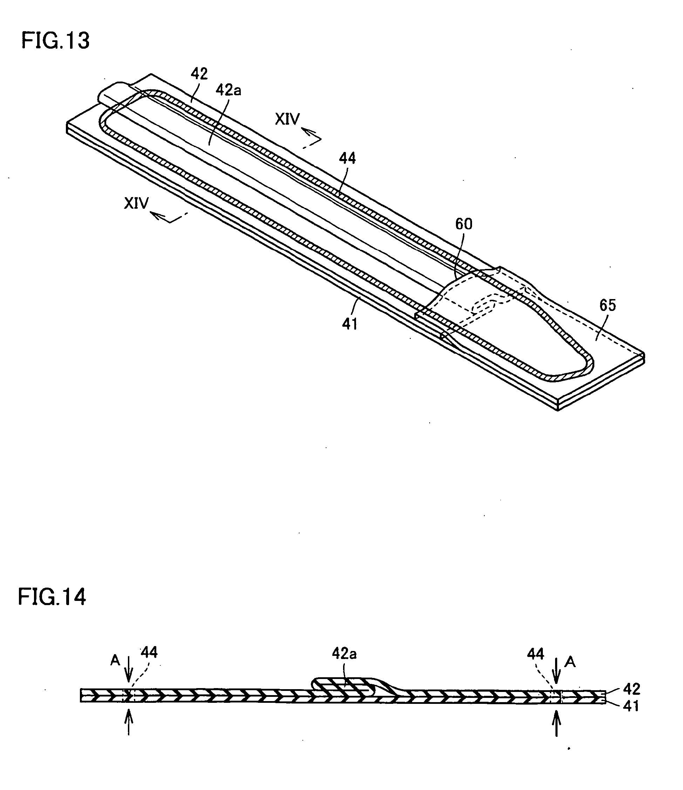

Fig. 13 is a schematic perspective view illustrating a process step in the other manufacturing method of the cuff for a blood pressure monitor according to Example 1 based on the embodiment of the present invention.

Fig. 14 is a schematic cross sectional view taken along the line XIV-XIV in Fig. 13.

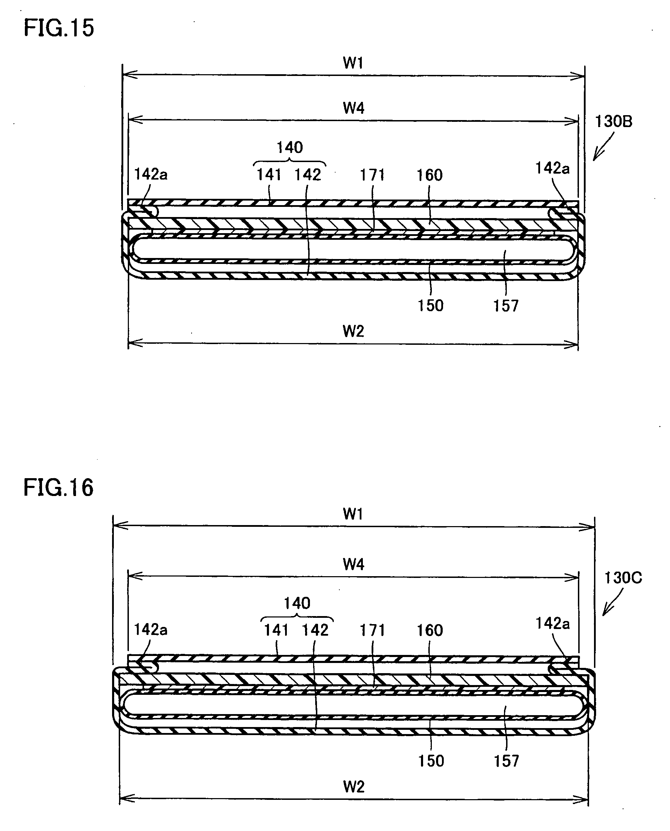

Fig. 15 is a schematic cross sectional view of a modification of the cuff for a blood pressure monitor according to Example 1 based on the embodiment of the present invention.

Fig. 16 is a schematic cross sectional view of another modification of the cuff for a blood pressure monitor according to Example 1 based on the embodiment of the present invention.

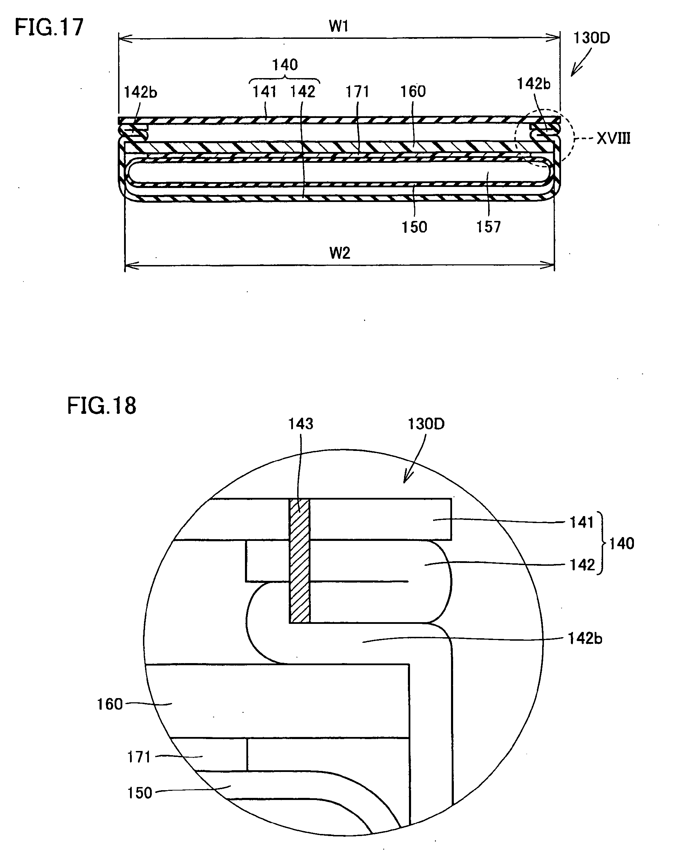

Fig. 17 is a schematic cross sectional view in the width direction of a cufffor a blood pressure monitor according to Example 2 based on the embodiment of the present invention.

Fig. 18 is an enlarged cross sectional view of a region XVIII, shown in Fig. 17, of the cuff for a blood pressure monitor according to Example 2 based on the embodiment of the present invention.

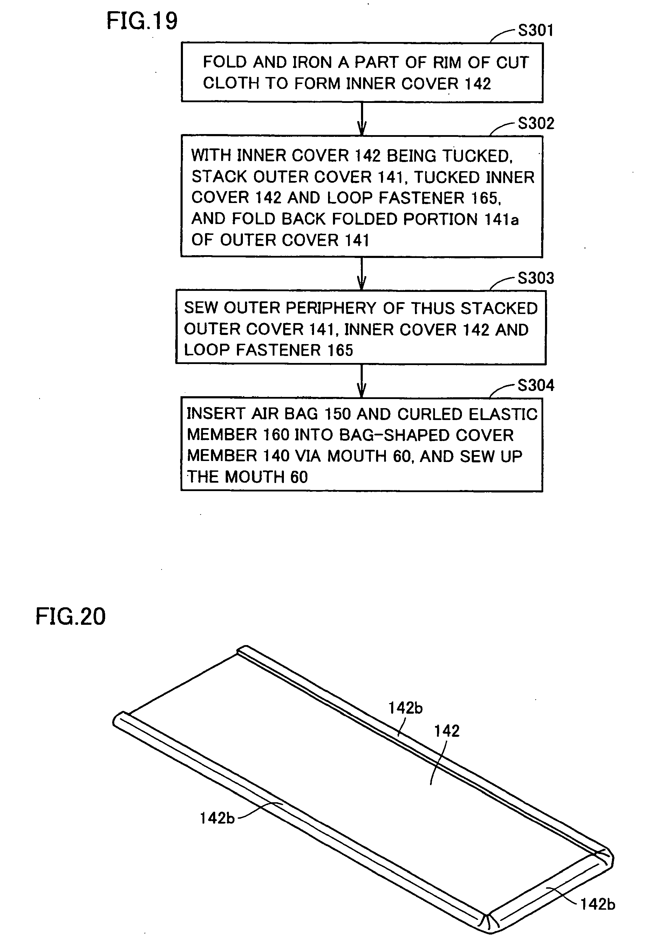

Fig. 19 is a flowchart illustrating a manufacturing method of the cuff for a blood pressure monitor according to Example 2 based on the embodiment of the present invention.

Fig. 20 is a schematic perspective view illustrating a process step in the manufacturing method of the cuff for a blood pressure monitor according to Example 2 based on the embodiment of the present invention.

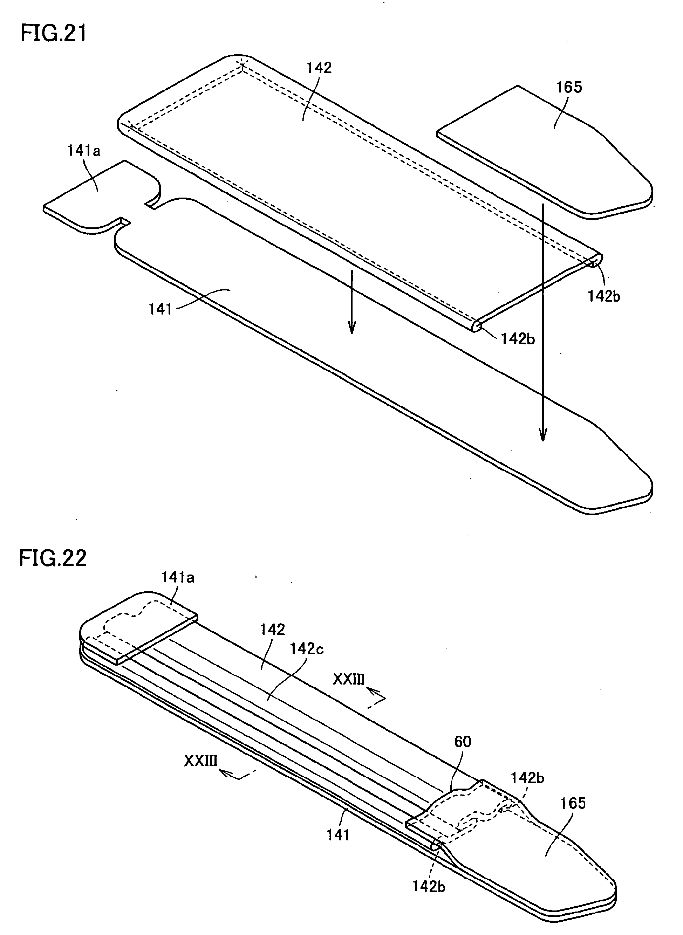

Fig. 21 is an exploded perspective view illustrating a process step in the manufacturing method of the cuff for a blood pressure monitor according to Example 2 based on the embodiment of the present invention.

Fig. 22 is a schematic perspective view illustrating a process step in the manufacturing method of the cuff for a blood pressure monitor according to Example 2 based on the embodiment of the present invention.

Fig. 23 is a schematic cross sectional view taken along the line XXIII-XXIII shown in Fig. 22.

Fig. 24 is a cross sectional view in the width direction of a cuff for a blood pressure monitor according to Conventional Example 1.

Fig. 25 is a cross sectional view in the width direction of a cuff for a blood pressure monitor according to Conventional Example 2.

Fig. 26 is a cross sectional view in the width direction of a cuff for a blood pressure monitor according to Conventional Example 3.

DESCRIPTION OF THE PREFERRED EMBODIMENTS

Hereinafter, an embodiment of the present invention will be described in detail with reference to the drawings. In the following embodiment, a wrist blood pressure monitor will be described as an example of the blood pressure monitor.

Fig. 1 is a perspective view of a blood pressure monitor according to the embodiment of the present invention. Fig. 2 is a vertical cross sectional view showing an inner structure of a cuff for the blood pressure monitor shown in Fig. 1. Firstly, the structure of the blood pressure monitor according to the embodiment of the present invention will be described with reference to Figs. 1 and 2.

As shown in Fig. 1, the blood pressure monitor 100 of the present embodiment includes a main body 110 and a cuff 130. A display portion 111 and a manipulation portion 112 are arranged on a surface of main body 110. Cuff 130 is attached to main body 110.

As shown in Fig. 2, cuff 130 for the blood pressure monitor primarily includes a bag-shaped cover member 140 that is made of cloth or the like, an air bag 150 identified as a fluid bag that is arranged inside bag-shaped cover member 140, and a curled elastic member 160 that is arranged inside bag-shaped cover member 140 on an outer side of air bag 150 in the fitted state of the cuff. Bag-shaped cover member 140, air bag 150 and curled elastic member 160 extend with their longitudinal direction corresponding to the winding direction of cuff 130.

Bag-shaped cover member 140 has an inner cover 142 constituting an inner wall portion located on the living body side, and an outer cover 141 constituting an outer wall portion located on the outer side than the inner wall portion. Inner cover 142 and outer cover 141 are stacked one on the other and their rims are connected to form a bag shape. On one end in the longitudinal direction of bag-shaped cover member 140, a loop fastener 165 is provided on the inner peripheral surface, to constitute the inner wall portion of bag-shaped cover member 140 together with inner cover 142. On the other end in the longitudinal direction of bag-shaped cover member 140, a hook fastener 166 is attached to the outer peripheral surface, for engagement with loop fastener 165. Hook and loop fasteners 166, 165 are members for securing blood pressure monitor 100 on the measurement site of the wrist in a stable manner when cuff 130 is mounted on the wrist.

Air bag 150 is made of a member of a bag shape that is formed, e.g., using resin sheets, and has an inflated/deflated space 157 therein. The outer surface on the wrist side of air bag 150 serves as a working face for pressing the wrist. Inflated/deflated space 157 is connected via a tube 120 to an air system 121 for blood pressure measurement in main body 110, which will be described later (see Fig. 3).

As the material for the resin sheets constituting air bag 150, any material can be used as long as it exhibits excellent elasticity and prevents leakage of the air from inflated/deflated space 157 after sealing. From these standpoints, optimal materials for the resin sheets include copolymer of ethylene-vinyl acetate (EVA), soft polyvinyl chloride (PVC), polyurethane (PU), crude rubber, and the like.

On the outer side of air bag 150, curled elastic member 160 identified as an elastic member is arranged, which is wound in an annular shape and elastically deformable in a radial direction. Curled elastic member 160 is attached to the outer peripheral surface of air bag 150 using an attaching member such as a double-faced tape (not shown). Curled elastic member 160 is configured to maintain its own annular shape corresponding to the contour of the wrist, and facilitates fitting of cuff 130 on the measurement site by the subject himself/herself. Curled elastic member 160 is made of a resin member of polypropylene or the like, so as to exert sufficient elastic force.

Hereinafter, functional blocks of the blood pressure monitor of the present embodiment will be described. Fig. 3 is a block diagram showing a configuration of the blood pressure monitor according to the present embodiment.

As shown in Fig. 3, main body 110 of blood pressure monitor 100 of the present embodiment includes an air system 121 for blood pressure measurement for supplying and evacuating the air to and from the above-described air bag 150 via a tube 120, and an oscillation circuit 125, a pump driving circuit 126 and a valve driving circuit 127 which are provided in association with air system 121 for blood pressure measurement. These components function as an inflating/deflating portion for inflating and deflating air bag 150.

Main body 110 further includes a CPU (Central Processing Unit) 113 for controlling and monitoring the respective portions in a centralized manner, a memory portion 114 for storing a program for causing CPU 113 to conduct a prescribed operation and various information including blood pressure values measured, a display portion 111 for displaying various information including a blood pressure measurement result, a manipulation portion 112 manipulated for inputting various instructions for measurement, and a power supply portion 115 for supplying electric power to CPU 113 by an instruction of power ON from manipulation portion 112. CPU 113 serves as a blood pressure value calculating portion for calculating a blood pressure value.

Air system 121 for blood pressure measurement has a pressure sensor 122 having an output value changed in accordance with the pressure within air bag 150 (hereinafter, referred to as "cuff pressure"), a pump 123 for supplying the air to air bag 150, and a valve 124 that is opened or closed to evacuate the air from or seal the air in air bag 150. Pressure sensor 122 serves as a pressure detecting portion for detecting the cuff pressure. Oscillation circuit 125 outputs to CPU 113 a signal of oscillation frequency corresponding to the output value of pressure sensor 122. Pump driving circuit 126 controls driving of pump 123 based on a control signal supplied from CPU 113. Valve driving circuit 127 controls opening/closing of valve 124 based on a control signal supplied from CPU 113.

Hereinafter, a process flow of blood pressure measurement by the blood pressure monitor of the present embodiment will be explained. Fig. 4 is a flowchart illustrating the process flow of blood pressure measurement by the blood pressure monitor according to the present embodiment. The program according to this flowchart is prestored in memory portion 114, and the blood pressure measuring process is carried out as CPU 113 reads out this program from memory portion 114 and executes the same.

As shown in Fig. 4, when a subject manipulates a manipulation button on manipulation portion 112 of blood pressure monitor 100 to turn the power ON, blood pressure monitor 100 is initialized (step S1). When it becomes a measurable state, CPU 113 starts driving of pump 123 to increase the cuff pressure of air bag 150 (step S2). During the process of increasing the pressure, when the cuff pressure reaches a prescribed level for measuring the blood pressure, CPU 113 stops pump 123, and gradually opens the closed valve 124 to evacuate the air from air bag 150, so as to gradually reduce the cuff pressure (step S3). In the present embodiment, the blood pressure is measured during the process of gradually decreasing the cuff pressure.

Next, CPU 113 calculates the blood pressure (systolic blood pressure, diastolic blood pressure) in a known manner (step S4). Specifically, during the process where the cuff pressure is gradually decreased, CPU 113 extracts pulse wave information based on the oscillation frequency obtained from oscillation circuit 125. It then calculates the blood pressure value from the pulse wave information extracted. The blood pressure value obtained in step S4 is displayed on display portion 111 (step S5). Although the measurement method described above is based on a so-called "decreasing-pressure measurement method" where the pulse waves are detected while the air bag is being decreased in pressure, it is of course possible to employ a so-called "increasing-pressure measurement method" where the pulse waves are detected while the air bag is being increased in pressure.

Blood pressure monitor 100 and cuff 130 incorporated in blood pressure monitor 100 of the embodiment described above are characterized by the structure of bag-shaped cover member 140 containing air bag 150 and curled elastic member 160 therein. Hereinafter, the structure of bag-shaped cover member 140 will be described in detail for each of Examples with reference to the drawings.

Example 1

Fig. 5 is a schematic cross sectional view of a cuff for a blood pressure monitor according to Example 1 based on the embodiment of the present invention, taken along the line V-V shown in Fig. 2. Fig. 6 is an enlarged cross sectional view of a region VI shown in Fig. 5. The structure of the cuff for a blood pressure monitor of the present example will now be described in detail with reference to Figs. 5 and 6.

As shown in Fig. 5, the cuff 130A for a blood pressure monitor according to the present example primarily includes an air bag 150 identified as a fluid bag that is inflated and deflated as the air identified as a fluid comes in and out, a curled elastic member 160 identified as an elastic member that is located on the outer side of air bag 150 in the state where air bag 150 is wound around a wrist, and a bag-shaped cover member 140 that contains air bag 150 and curled elastic member 160 therein, as described above. Air bag 150 has its outer peripheral surface fixed to curled elastic member 160 via an attaching member such as a double-faced tape 171, to make air bag 150 and curled elastic member 160 integrated with each other.

Bag-shaped cover member 140 includes an inner cover 142 made of highly elastic cloth or the like and located on an inner side in the fitted state of the cuff, and an outer cover 141 made of less elastic cloth or the like and located on the outer side of inner cover 142. Bag-shaped cover member 140 is formed into a bag shape by stacking inner cover 142 and outer cover 141 one on the other and connecting their rims together. Each of inner cover 142 and outer cover 141 may be formed of a plurality of sheet-shaped members if necessary, instead of being formed of a single sheet-shaped member. For example, a plurality of sheet-shaped members may be joined edge to edge in the longitudinal direction to form each of inner and outer covers 142 and 141, or a plurality of sheet-shaped members may be stacked one on another in the thickness direction to form each of inner and outer covers 142 and 141.

As shown in Fig. 5, in cuff 130A for a blood pressure monitor of the present example, inner cover 142 extending in the longitudinal direction has a length in its width direction (orthogonal to the longitudinal direction) longer than that of outer cover 141. That is, inner cover 142 and outer cover 141 are connected together, with certain slack allowed in inner cover 142,

Inner cover 142 has a folded portion 142a at each end in the width direction that is formed by folding its end portion. Folded portion 142a is located between the end portion in the width direction of curled elastic member 160 contained in bag-shaped cover member 140 and the end portion in the width direction of outer cover 141. More specifically, as shown in Fig. 6, assuming that the vertical direction in the figure corresponds to the vertical direction of cuff 130A for a blood pressure monitor, two sheet-shaped members constituting inner and outer covers 142 and 141 are sewn together with a thread, for example, in the state where they are stacked one on the other such that an upper surface of the end portion in the width direction of inner cover 142 faces a lower surface of the end portion in the width direction of outer cover 141 over a prescribed region including their edges. As a result, a sewn portion 143 is formed at a position a prescribed distance inward from the edges, and inner cover 142 is folded back outward in the width direction at sewn portion 143. Inner cover 142 is further bent at an approximately right angle along the end portion in the width direction of curled elastic member 60. Accordingly, folded portion 142a is arranged between the end portion of outer cover 141 and the end portion of curled elastic member 160.

With this configuration, sewn portion 143 identified as a connected portion of outer cover 141 and inner cover 142 is located inner than each end portion in the width direction of cuff 130A for a blood pressure monitor, as shown in Fig. 5, and accordingly, it is possible to make a size W2 in the width direction of air bag 150 sufficiently large with respect to a width dimension W1 of cuff 130A for a blood pressure monitor. The size of air bag 150 in the width direction can be increased to substantially correspond to the width dimension of cuff 130A for a blood pressure monitor. Thus, even if the cuff is reduced in width, the artery can be pressed for avascularization in a stable manner along the axial direction of the wrist, and therefore, it is possible to accurately and stably measure the blood pressure values.

Further, as shown in Fig. 5, there is no unlevel portion on the outer peripheral surface of cuff 130A for a blood pressure monitor, which eliminates the undesired situation where the finger or the like would be caught and cause curling or fraying of bag-shaped cover member 140. As such, a highly reliable cuff for a blood pressure monitor excellent in handling property can be obtained. Still further, since there is no unlevel portion on the outer peripheral surface that would otherwise adversely affect the appearance of the product, good appearance of the cuff for a blood pressure monitor can be maintained.

Fig. 7 is a schematic cross sectional view showing another configuration of the connected portion of the cuff for a blood pressure monitor according to the present example. In the foregoing, explanation was made about the case where inner cover 142 and outer cover 141 are connected via sewing. Alternatively, inner cover 142 and outer cover 141 may be connected together at their rims by melting and bonding with high frequency wave, as shown, e.g., in Fig. 7.

In this case, as shown in Fig. 7, when the vertical direction in the figure corresponds to the vertical direction of cuff 130A for a blood pressure monitor, an upper surface of the end portion in the width direction of inner cover 142 and a lower surface of the end portion in the width direction of outer cover 141 are melted and bonded together over a prescribed region including their edges, so that a bonded portion 144 is formed which extends inward from the edges for a prescribed distance. Inner cover 142 is then folded back outward in the width direction at the inner end portion of bonded portion 144, and inner cover 142 is further bent at an approximately right angle along the end portion in the width direction of curled elastic member 160. As such, folded portion 142a is arranged between the end portion of outer cover 141 and the end portion of curled elastic member 160.

It should be understood that it is of course possible to combine the connection by melting and bonding and the connection by sewing as described above to connect inner cover 142 and outer cover 141.

Hereinafter, a specific method for manufacturing cuff 130A for a blood pressure monitor according to the present example will be described. Fig. 8 is a flowchart illustrating a manufacturing method of the cuff for a blood pressure monitor according to the present example. Figs. 9 and 10 are schematic perspective views illustrating the manufacturing method shown in Fig. 8. Specifically, Fig. 9 shows the step of stacking cloths and the like one on another, and Fig. 10 shows the step of connecting them. The manufacturing method of the cuff for a blood pressure monitor according to the present example shows, by way of example, the case of connecting inner cover 142 and outer cover 141 by a combination of melting and bonding with high frequency wave and sewing.

As shown in Fig. 8, to manufacture cuff 130A for a blood pressure monitor of the configuration described above, firstly in step S101, a cloth 41 to be outer cover 141 and a cloth 42 to be inner cover 142 and a loop fastener 65 are stacked one on another. At this time, a spacer member 50 of a prescribed size is interposed between cloth 41 and cloth 42. Although the size of spacer member 50 is determined in accordance with the size of cuff 130A for a blood pressure monitor to be manufactured, it should be at least narrower in width than cuff 130A for a blood pressure monitor and have a prescribed thickness. Cloths 41, 42 and loop fastener 65 are stacked such that cloth 42 and loop fastener 65 partially overlap each other in the longitudinal direction, and that cloth 42 is sandwiched between loop fastener 65 and cloth 41 at the overlapped portion.

Next, as shown in Fig. 8, in step S 102, cloths 41, 42 and loop fastener 65 thus stacked are temporarily connected by melting and bonding with high frequency wave, and cut along the outer periphery of the bonded portion. More specifically, as shown in Fig. 10, with spacer member 50 interposed between stacked cloths 41 and 42, cloths 41, 42 and loop fastener 65 are connected by melting and bonding with high frequency wave. At this time, the portion to be melted and bonded is set to surround spacer member 50 in accordance with the pre-designed outer shape of cuff 130A for a blood pressure monitor. The melting and bonding is carried out across the overlapped portion of cloth 42 and loop fastener 65. As such, cloth 42 to be inner cover 142 and cloth 41 to be outer cover 141 are melted and bonded only in a selected region of the rims of inner and outer covers 142 and 141, and an opening is formed between cloth 42 and loop fastener 65 in the non-selected region, which extends in the width direction. This opening serves as a mouth 60 through which the spacer member is taken out and an air bag 150 and a curled elastic member 160 are inserted, as will be described later. Thereafter, the excess portions of cloths 41, 42 and loop fastener 65 are cut along the outer periphery of bonded portion 44 formed by the above-described melting and bonding process. In this manner, cloths 41, 42 and loop fastener 65 are cut into the outer shapes of outer cover 141, inner cover 142 and loop fastener 165 as explained above.

Next, as shown in Fig. 8, in step S103, spacer member 50 having been arranged inside bag-shaped cover member 140 is taken out through mouth 60. In step S 104, cloth 41 to be outer cover 141, cloth 42 to be inner cover 142 and loop fastener 65 are sewn along bonded portion 44 for actual connection thereof. Then, in step S 105, air bag 150 and curled elastic member 160 having been integrated with each other via an attaching member such as a double-faced tape are inserted into bag-shaped cover member 140 through mouth 60. Mouth 60 is then sewn up to complete cuff 130A for a blood pressure monitor of the above-described configuration.

When the manufacturing method as described above is employed, spacer member 50 is sandwiched between cloths 41 and 42 when stacking them. In this state, the rims of cloths 41 and 42 are connected together. The presence of spacer member 50 results in formation of slack in cloth 42 that is more elastic than cloth 41. This slack is adjusted to have a size necessary for forming the above-described folded portion 142a at inner cover 142. Thus, after spacer member 50 is taken out and after air bag 150 and curled elastic member 160 are inserted, folded portions 142a of inner cover 142 are formed between the respective end portions in the width direction of curled elastic member 160 and the corresponding end portions in the width direction of outer cover 141.

Thus, by employing this manufacturing method, the slack can readily be formed at inner cover 142. Accordingly, it is possible to manufacture cuff 130A for a blood pressure monitor having folded portion 142a of inner cover 142 arranged between the end portion in the width direction of outer cover 141 and the end portion in the width direction of curled elastic member 160, inexpensively and with good workability.

Cuff 130A for a blood pressure monitor according to the present example may be manufactured using another manufacturing method. Hereinafter, another specific example of the manufacturing method of cuff 130A for a blood pressure monitor of the present example will be described. Fig. 11 is a flowchart illustrating another manufacturing method for manufacturing the cuff for a blood pressure monitor according to the present example. Figs. 12 and 13 are schematic perspective views illustrating the manufacturing method of the cuff for a blood pressure monitor shown in Fig. 11. Specifically, Fig. 12 shows the step of stacking the cloths and the like, and Fig. 13 shows the step of connecting them. Further, Fig. 14 is a schematic cross sectional view taken along the line XIV-XIV shown in Fig. 13. The manufacturing method of the cuff for a blood pressure monitor of the present example involves high-frequency melting and bonding and sewing for connecting inner and outer covers 142 and 141, as in the case of the manufacturing method of the cuff for a blood pressure monitor described above.

As shown in Fig. 11, firstly in step S201, cloth 41 to be outer cover 141, cloth 42 to be inner cover 142 and loop fastener 65 are stacked one on another. At this time, as shown in Fig. 12, cloth 42 to be inner cover 142 is made to have a width greater than that of cloth 41 to be outer cover 141. Upon stacking cloths 41 and 42, a part of cloth 42 is tucked to form a tucked portion 42a that extends along the direction crossing the width direction of cloth 42, and cloths 41 and 42 are stacked with their end portions in the width direction aligned with each other. Cloth 42 and loop fastener 65 are stacked such that they partially overlap each other in the longitudinal direction, and cloth 42 is sandwiched between loop fastener 65 and cloth 41 at the overlapped portion.

Next, as shown in Fig. 11, in step S202, cloths 41, 42 and loop fastener 65 thus stacked are temporarily connected by melting and bonding with high frequency wave, and cut along the outer periphery of the bonded portion. More specifically, as shown in Figs. 13 and 14, cloths 41, 42 and loop fastener 65, stacked in the state where a prescribed part of cloth 42 has been tucked to form tucked portion 42a, are connected by melting and bonding with high frequency wave. At this time, the portion to be melted and bonded (the portion shown in an arrow A in Fig. 14) is set in accordance with the pre-designed outer shape of cuff 130A for a blood pressure monitor. The melting and bonding is carried out across the overlapped portion of cloth 42 and loop fastener 65. As such, the melting and bonding of cloth 42 to be inner cover 142 and cloth 41 to be outer cover 141 is carried out only in a selected region of the rims of inner and outer covers 142 and 141, and an opening is formed between cloth 42 and loop fastener 65 in the non-selected region, to extend in the width direction. This opening serves as a mouth 60 through which air bag 150 and curled elastic member 160 will be inserted, as will be described later. Thereafter, the excess portions of cloths 41, 42 and loop fastener 65 are cut along the outer periphery of the bonded portion 44 formed by the above-described melting and bonding process. In this manner, cloths 41, 42 and loop fastener 65 are cut into the outer shapes of outer cover 141, inner cover 142 and loop fastener 165 described above.

Next, as shown in Fig. 11, in step S203, cloth 41 to be outer cover 141, cloth 42 to be inner cover 142 and loop fastener 65 are sewn along bonded portion 44 for actual connection thereof. Then, in step S204, air bag 150 and curled elastic member 160 having been integrated with each other via an attaching member such as a double-faced tape are inserted into bag-shaped cover member 140 through mouth 60. Thereafter, mouth 60 is sewn up to complete cuff 130A for a blood pressure monitor of the configuration described above.

When the above-described manufacturing method is employed, upon stacking of cloths 41 and 42, cloth 42 is tucked at a portion other than the end portion in the width direction to form tucked portion 42a that extends along the direction crossing the width direction. In this state, the rims of cloths 41 and 42 are connected together. With the presence of tucked portion 42a, slack is formed at cloth 42 that is more elastic than cloth 41. This slack is adjusted to have a size necessary for forming the above-described folded portion 142a of inner cover 142. Thus, after insertion of air bag 150 and curled elastic member 160, folded portions 142a of inner cover 142 are formed between the respective end portions in the width direction of curled elastic member 160 and the corresponding end portions in the width direction of outer cover 141.

Thus, by employing this manufacturing method, the slack is readily formed at inner cover 142. Therefore, it is possible to form a cuff for a blood pressure monitor having folded portion 142a of inner cover 142 arranged between the end portion in the width direction of outer cover 141 and the end portion in the width direction of curled elastic member 160, inexpensively and with good workability. Although the case of forming one tucked portion 42a in cloth 42 has been described above, a plurality of such tucked portions may be provided in the width direction of cloth 42 as required.

In cuff 130A for a blood pressure monitor of the present example described above, the size in the width direction of inner cover 142 is made greater than the size in the width direction of outer cover 141. Here, the size in the width direction of inner cover 142 is preferably calculated based on the following expression:

$$(\text{size in width direction of inner cover}) = [(\text{size in width direction of outer cover}) + \{(\text{thickness of insert such as air bag and curled elastic member}) + (\text{length of folded portion of inner cover})\} \times 2] \pm \text{margin for fine adjustment}.$$

By setting the length in the width direction of inner cover 142 based on the above expression, inner cover 142 will not suffer excessive tension, and unnecessarily large slack will not be formed in inner cover 142. Thus, bag-shaped cover member 140 suitable when manufacturing the cuff for a blood pressure monitor of the present example can be provided.

Next, a modification of the cuff for a blood pressure monitor according to Example 1 described above will be explained. Fig. 15 is a schematic cross sectional view of a modification of the cuff for a blood pressure monitor of Example 1. Fig. 16 is a schematic cross sectional view of another modification of the cuff for a blood pressure monitor of Example 1. In the drawings, the portions similar to those of the cuff for a blood pressure monitor of Example 1 have the same reference characters allotted, and description thereof will not be repeated.

In cuff 130A for a blood pressure monitor of Example 1 described above, the size in the width direction of air bag 150 is made smaller than the size in the width direction of outer cover 141 by two folds of the thickness of inner cover 142, to make width dimension W1 of cuff 130A for a blood pressure monitor correspond to that of outer cover 141. As such, width dimension W1 of cuff 130A for a blood pressure monitor is substantially equal to size W2 in the width direction of air bag 150 (see Fig. 5).

By comparison, in the modification shown in Fig. 15, a size W4 in the width direction of outer cover 141 is made equal to size W2 in the width direction of air bag 150. With this configuration, although size W2 in the width direction of air bag 150 is smaller than width dimension W1 of cuff 130B for a blood pressure monitor by two folds of the thickness of inner cover 142, as in the case of cuff 130A for a blood pressure monitor of Example 1, in cuff 130B for a blood pressure monitor of the present modification, size W2 in the width direction of air bag 150 can be made equal to size W4 in the width direction of outer cover 141, so that the cuff for a blood pressure monitor narrower in appearance can be obtained.

Further, in the modification shown in Fig. 16, size W2 in the width direction of air bag 150 is made greater than size W4 in the width direction of outer cover 141. With this configuration, although size W2 in the width direction of air bag 150 is smaller than width dimension W1 of cuff 130C for a blood pressure monitor by two folds of the thickness of inner cover 142, again as in the case of cuff 130A for a blood pressure monitor of Example 1, in cuff 130C for a blood pressure monitor of the present modification, size W2 in the width direction of air bag 150 can be made greater than size W4 in the width direction of outer cover 141, so that the cuff for a blood pressure monitor narrower in appearance can be obtained.

Example 2

Fig. 17 is a schematic cross sectional view in a width direction of a cuff for a blood pressure monitor according to Example 2 based on the present embodiment. Fig. 18 is an enlarged cross sectional view of a region XVIII shown in Fig. 17. Hereinafter, a structure of the cuff for a blood pressure monitor according to the present example will be described in detail with reference to Figs. 17 and 18. The portions identical to those of cuff 130A for a blood pressure monitor of Example 1 described above have the same reference characters allotted, and description thereof will not be repeated.

In cuff 130A for a blood pressure monitor according to Example 1 described above, inner cover 142 has its edge at each end portion in the width direction facing the outside of cuff 130A for a blood pressure monitor. Inner cover 142 is preferably formed of cloth more elastic than that of outer cover 141, which means that it is often made of relatively thin cloth. Thus, when the edge at each end portion in the width direction of inner cover 142 is exposed to the outside as described above, inner cover 142 may suffer fraying at the relevant portion after long-term use.

Thus, in the cuff 130D for a blood pressure monitor of the present example, the edge at each end portion in the width direction of inner cover 142 is further folded inward, as shown in Figs. 17 and 18, so that the edge is hidden inside cuff 130D for a blood pressure monitor. More specifically, the folded portion 142b located at each end portion of inner cover 142 is double folded, such that inner cover 142 is triple-layered at the end portion. This prevents the edge at each end portion in the width direction of inner cover 142 from being exposed to the outside of cuff 130D for a blood pressure monitor, so that occurrence of fraying is suppressed.

Hereinafter, a specific method for manufacturing cuff 130D for a blood pressure monitor according to the present example will be described. Fig. 19 is a flowchart illustrating the manufacturing method of the cuff for a blood pressure monitor of the present example. Figs. 20-22 are schematic perspective views illustrating the manufacturing method shown in Fig. 19. Specifically, Fig. 20 shows the step of folding the end portion of the inner cover, Fig. 21 shows the step of stacking the inner and outer covers and the loop fastener, and Fig. 22 shows the step of connecting them. Further, Fig. 23 is a schematic cross sectional view taken along the line XXIII-XXIII shown in Fig. 22. The manufacturing method of the cuff for a blood pressure monitor of the present example shows, by way of example, the case of connecting inner cover 142 and outer cover 141 by sewing.

As shown in Fig. 19, firstly in step S301, long sides and one short side of an inner cover 142 cut out into an approximately rectangular shape are folded inward by a prescribed amount and ironed, for example, to form a folded portion 142b (see Fig. 20).

Next, in step S302, inner cover 142 and a loop fastener 165 are stacked on an outer cover 141 cut out into a prescribed shape. At this time, for inner cover 142, the one having been shaped in step S301 is used, as shown in Fig. 21. Inner cover 142 prepared is greater in width than outer cover 141. A part of inner cover 142 is tucked to form a tucked portion 142c extending along the direction crossing the width direction of inner cover 142, as shown in Fig. 22, and outer cover 141 and inner cover 142 are stacked with their end portions in the width direction aligned with each other. For outer cover 141, one having a folded portion 14 1 a at one end portion in the longitudinal direction is prepared. This folded portion 141 a is for covering the end portion of bag-shaped cover member 140 after formation thereof, so as to guarantee good appearance of the end portion of bag-shaped cover member 140 and also to prevent occurrence of curling or fraying of the relevant portion. Furthermore, the edge of curled elastic member 160 can be fitted into a pocket-shaped portion created between bag-shaped cover member 140 and folded portion 141a to improve the fitting property. Inner cover 142 and loop fastener 165 are stacked such that they partially overlap each other in the longitudinal direction, and inner cover 142 is made to be sandwiched between loop fastener 165 and outer cover 141 at the overlapped portion.

Next, as shown in Fig. 19, in step S303, outer cover 141, inner cover 142 and loop fastener 165 thus stacked are sewn together. More specifically, as shown in Fig. 22, with a prescribed portion of inner cover 142 being tucked to form tucked portion 142c, outer cover 141, inner cover 142 and loop fastener 165 are sewn together. Before sewing, folded portion 141a provided in advance at one end in the longitudinal direction of outer cover 141 is folded back at cut portions to thereby cover the end portion in the longitudinal direction of inner cover 142. The portion to be sewn (shown by an arrow B in Fig. 23) is set along the outer periphery of the stack of outer cover 141, inner cover 142 and loop fastener 165. The sewing is carried out across the overlapped portion of inner cover 142 and loop fastener 165, whereby an opening is formed between inner cover 142 and loop fastener 165 to extend in the width direction. This opening serves as a mouth 60 through which air bag 150 and curled elastic member 160 are inserted, as will be described later.

Next, as shown in Fig. 19, in step S304, air bag 150 and curled elastic member 160 having been integrated with each other via an attaching member such as a double-faced tape are inserted into bag-shaped cover member 140 via mouth 60. Mouth 60 is then sewn up to complete cuff 130D for a blood pressure monitor configured as described above.

When the above-described manufacturing method is employed, cuff 130D for a blood pressure monitor with the structure where inner cover 142 has its edge at each end portion in the width direction hidden inside can readily be obtained.

In the embodiment of the present invention described above, the case of producing the bag-shaped cover member using cloths has been explained. The material for the bag-shaped cover member however is not restricted to cloth. Further, in the embodiment described above, the case of attaching the curled elastic member to the air bag using the double-faced tape has been explained. However, they are not necessarily fixed to each other by adhesion or the like. They may be fixed using another method, or they may be left completely unfixed with each other.

Further, in the embodiment described above, the case of applying the present invention to a cuff for use in a wrist blood pressure monitor assuming the wrist as the measurement site has been explained. However, not limited thereto, the present invention is applicable to a cuff for any type of blood pressure monitor, including an upper arm type and a finger type.

Although the present invention has been described and illustrated in detail, it is clearly understood that the same is by way of illustration and example only and is not to be taken by way of limitation, the scope of the present invention being limited only by the terms of the appended claims.

## Claims

1. A cuff (130) for a blood pressure monitor (100), comprising:

> a fluid bag (150) inflated and deflated as a fluid comes in and out;
> an elastic member (160) located on an outer side of said fluid bag in the state where said fluid bag is wound around a living body, and wound annularly and changeable in size in a radial direction; and
> a bag-shaped cover member (140) containing said fluid bag and said elastic member therein; wherein
> said bag-shaped cover member (140) includes an inner cover (142) forming an inner wall portion located on a living body side when the bag-shaped cover member is wound around the living body, and an outer cover (141)

forming an outer wall portion located on an outer side than said inner wall portion in the state where the bag-shaped cover member is wound around the living body, the inner and outer covers being stacked one on the other and having their rims connected together to form a bag shape,
said inner cover (142) has end portions in its width direction each folded back to form a folded portion (142a), and said folded portion is located between an end portion in a width direction of said outer cover (141) and an end portion in a width direction of said elastic member (160), **characterized in that**
said folded portion (142a) includes at least a portion folded back outwardly in the width direction.

2. The cuff for a blood pressure monitor according to claim 1, wherein said inner cover (142) is more elastic than said outer cover (141).

3. The cuff for a blood pressure monitor according to claim 1, wherein in a deflated state where said fluid bag (150) is not pressurized, said fluid bag has a size in its width direction substantially equal to a width dimension of the cuff (130) for a blood pressure monitor.

4. A blood pressure monitor (100), comprising:

   a cuff (130) as defined in claim 1;
   an inflating/deflating portion (123, 124) for inflating and deflating said fluid bag;
   a pressure detecting portion (122) for detecting a pressure within said fluid bag; and
   a blood pressure value calculating portion (113) for calculating a blood pressure value based on pressure information detected by said pressure detecting portion

5. The blood pressure monitor according to claim 4, wherein said inner cover (142) is more elastic than said outer cover (141).

6. The blood pressure monitor according to claim 4, wherein in a deflated state where said fluid bag (150) is not pressurized, said fluid bag has a size in its width direction substantially equal to a width dimension of said cuff (130) for a blood pressure monitor.

7. A manufacturing method of a cuff (130) according to any of claims 1 to 3,
   the method comprising the steps of:

   stacking said inner cover (142) and said outer cover (141) with a spacer member (50) interposed therebetween, and connecting the rims of said inner and outer covers in a selected region to allow slack sufficient to form said folded portion in said inner cover;
   taking the spacer member (50) out of said bag-shaped cover member (140); and
   inserting said fluid bag and said elastic member into said bag-shaped cover member (140) provided with the slack to thereby form said folded portion (142) at said inner cover.

8. A manufacturing method of a cuff (130) according to any of claims 1 to 3,
   the method comprising the steps of:

   stacking said inner cover (142) and said outer cover (141) in the state where said inner cover is tucked at a portion other than its end portion to reduce a width of said inner cover, and connecting the rims of said inner and outer covers in a selected region to allow slack sufficient to form said folded portion in said inner cover; and
   inserting said fluid bag (150) and said elastic member (160) into said bag-shaped cover member (140) provided with the slack to thereby form said folded portion at said inner cover.

**Patentansprüche**

1. Manschette (130) für ein Blutdruckmessgerät (100), welche aufweist:

   einen Fluidsack (150) der mit dem Ein- und Austreten von Fluid aufgepumpt und entleert wird;
   ein elastisches Element (160), welches in dem Zustand, in dem der Fluidsack um einen Lebewesenkörper gelegt ist, an einer Außenseite des Fluidsacks angeordnet ist und welches ringförmig gewickelt und in einer radialen Richtung in der Größe änderbar ist; und

ein sackförmiges Abdeckelement (140), welches den Fluidsack und das elastische Element darin enthält; wobei das sackförmige Abdeckelement (140) eine innere Abdeckung (142), welche einen inneren Wandabschnitt bildet, der auf der Lebewesenkörperseite angeordnet ist, wenn das sackförmige Abdeckelement um den Lebewesenkörper gelegt ist, und eine äußere Abdeckung (141), die einen äußeren Wandabschnitt bildet, der an einer weiter außen als der innere Wandabschnitt liegenden Seite, in dem Zustand, in dem das sackförmige Abdeckelement um den Lebewesenkörper gelegt ist, liegt, enthält, wobei innere und äußere Abdeckung übereinander gelegt und mit ihren Rändern zu einer Sackform verbunden sind,

die innere Abdeckung (142) Endabschnitte in ihrer Breitenrichtung aufweist, die jeweils zur Bildung eines gefalteten Abschnitts (142a) zurückgefaltet sind, und

der gefaltete Abschnitt zwischen einem Endabschnitt in einer Breitenrichtung der äußeren Abdeckung (141) und einem Endabschnitt in der Breitenrichtung des elastischen Elements (160) angeordnet ist, **dadurch gekennzeichnet, dass**

der gefaltete Abschnitt (142a) wenigstens einen Abschnitt enthält, der nach außen in der Breitenrichtung zurückgefaltet ist.

2. Manschette für ein Blutdruckmessgerät nach Anspruch 1, wobei die innere Abdeckung (142) elastischer als die äußere Abdeckung (141) ist.

3. Manschette für ein Blutdruckmessgerät nach Anspruch 1, wobei in einem entleerten Zustand, in dem der Fluidsack (150) nicht unter Druck steht, der Fluidsack in seiner Breitenrichtung eine Größe hat, die im Wesentlichen gleich einer Breitenabmessung der Manschette (130) für ein Blutdruckmessgerät ist.

4. Blutdruckmessgerät (100), welches aufweist:

eine Manschette (130) wie in Anspruch 1 definiert;
einen Aufpump-/Entleerabschnitt (123, 124) zum Aufpumpen und Entleeren des Fluidsacks;
einen Drucknachweisabschnitt (122) zur Feststellung eines Drucks innerhalb des Fluidsacks; und
einen Blutdruckwertberechnungsabschnitt (113) zur Berechnung eines Blutdruckwerts beruhend auf mit dem Drucknachweisabschnitt festgestellter Druckinformation.

5. Blutdruckmessgerät nach Anspruch 4, wobei die innere Abdeckung (142) elastischer als die äußere Abdeckung (141) ist.

6. Blutdruckmessgerät nach Anspruch 4, wobei in einem entleerten Zustand, in dem der Fluidsack (150) nicht unter Druck steht, der Fluidsack in seiner Breitenrichtung eine Größe hat, die im Wesentlichen gleich einer Breitenabmessung der Manschette (130) für ein Blutdruckmessgerät ist.

7. Herstellungsverfahren für eine Manschette (130) nach einem der Ansprüche 1 bis 3,
wobei das Verfahren folgende Verfahrensschritte aufweist:

Übereinanderlegen der inneren Abdeckung (142) und der äußeren Abdeckung (141) unter Zwischenlage eines Abstandsteils (50) und Verbinden der Ränder von innerer und äußerer Abdeckung in einem ausgewählten Bereich so, dass ausreichend Lose zur Ausbildung des gefalteten Abschnitts in der inneren Abdeckung verbleibt; Herausnehmen des Abstandteils (50) aus dem sackförmigen Abdeckelement (140); und
Einsetzen des Fluidsacks und des elastischen Elements in das mit Lose vorgesehene sackförmige Abdeckelement (140), um so den gefalteten Abschnitt (142) an der inneren Abdeckung auszubilden.

8. Herstellungsverfahren für eine Manschette (130) nach einem der Ansprüche 1 bis 3,
wobei das Verfahren folgende Schritte aufweist:

Übereinanderlegen der inneren Abdeckung (142) und der äußeren Abdeckung (141) in dem Zustand, in dem die innere Abdeckung an einem von ihrem Endabschnitt verschiedenen Abschnitt zur Verminderung einer Breite der inneren Abdeckung gefaltet ist, und Verbinden der Ränder von innerer und äußerer Abdeckung in einem ausgewählten Bereich so, dass ausreichend Lose zur Ausbildung des gefalteten Abschnitts in der inneren Abdeckung verbleibt; und
Einsetzen des Fluidsacks (150) und des elastischen Elements (160) in das mit der Lose vorgesehene sackförmige Abdeckelement (140), um so den gefalteten Abschnitt an der inneren Abdeckung auszubilden.

**Revendications**

1. Brassard (130) pour tensiomètre (100), comprenant :

   un sac de fluide (150) gonflé et dégonflé lorsque le fluide rentre et sort ;
   un élément élastique (160) situé sur un côté externe dudit sac de fluide dans l'état où ledit sac de fluide est enroulé autour d'un corps vivant, et enroulé de façon annulaire et de taille variable dans une direction radiale ; et
   un élément d'enveloppe en forme de sac (140) contenant ledit sac de fluide et ledit élément élastique à l'intérieur ; dans lequel
   ledit élément d'enveloppe en forme de sac (140) comprend une enveloppe interne (142) formant une partie de paroi interne située sur un côté de corps vivant lorsque l'élément d'enveloppe en forme de sac est enroulé autour du corps vivant, et une enveloppe externe (141) formant une partie de paroi externe située sur un côté externe par rapport à ladite partie de paroi interne dans l'état où l'élément d'enveloppe en forme de sac est enroulé autour du corps vivant, les enveloppes interne et externe étant empilées l'une sur l'autre et ayant leurs bords reliés pour prendre une forme de sac,
   ladite enveloppe interne (142) a des parties d'extrémité dans la direction de la largeur repliées chacune pour former une partie pliée (142a), et
   ladite partie pliée est située entre une partie d'extrémité dans une direction de la largeur de ladite enveloppe externe (141) et une partie d'extrémité dans une direction de la largeur dudit élément élastique (160), **caractérisé en ce que**
   ladite partie pliée (142a) comprend au moins une partie repliée vers l'extérieur dans la direction de la largeur.

2. Brassard pour tensiomètre selon la revendication 1, dans lequel ladite enveloppe interne (142) est plus élastique que ladite enveloppe externe (141).

3. Brassard pour tensiomètre selon la revendication 1, dans lequel dans un état dégonflé dans lequel ledit sac de fluide (150) n'est pas pressurisé, ledit sac de fluide a une dimension dans la direction de la largeur sensiblement égale à une dimension de largeur du brassard (130) pour tensiomètre.

4. Tensiomètre (100), comprenant :

   un brassard (130) selon la revendication 1 ;
   une partie qui se gonfle/se dégonfle (123, 124) pour gonfler et dégonfler ledit sac de fluide ;
   une partie de détection de pression (122) pour détecter une pression à l'intérieur dudit sac de fluide ; et
   une partie de calcul de valeur de pression artérielle (113) pour calculer une valeur de pression artérielle sur la base des informations de pression détectées par ladite partie de détection de pression.

5. Tensiomètre selon la revendication 4, dans lequel ladite enveloppe interne (142) est plus élastique que ladite enveloppe externe (141).

6. Tensiomètre selon la revendication 4, dans lequel dans un état dégonflé dans lequel ledit sac de fluide (150) n'est pas pressurisé, ledit sac de fluide a une dimension dans la direction de la largeur sensiblement égale à une dimension de largeur dudit brassard (130) pour tensiomètre.

7. Procédé de fabrication d'un brassard (130) selon l'une quelconque des revendications 1 à 3,
   Le procédé comprenant les étapes consistant à :

   empiler ladite enveloppe interne (142) et ladite enveloppe externe (141) avec un élément d'écartement (50) intercalé entre elles, et relier les bords desdites enveloppes interne et externe dans une région sélectionnée pour offrir un jeu suffisant pour former ladite partie pliée dans ladite enveloppe interne ;
   sortir l'élément d'écartement (50) dudit élément d'enveloppe en forme de sac (140) ; et
   insérer ledit sac de fluide et ledit élément élastique dans ledit élément d'enveloppe en forme de sac (140) présentant le jeu pour former ainsi ladite partie pliée (142) au niveau de ladite enveloppe interne.

8. Procédé de fabrication d'un brassard (130) selon l'une quelconque des revendications 1 à 3,
   Le procédé comprenant en outre les étapes consistant à :

   empiler ladite enveloppe interne (142) et ladite enveloppe externe (141) dans l'état dans lequel ladite enveloppe interne est plissée au niveau d'une partie autre que sa partie d'extrémité pour réduire une largeur de ladite enveloppe interne, et relier les bords desdites enveloppes interne et externe dans une région sélectionnée pour offrir un jeu suffisant pour former ladite partie pliée dans ladite enveloppe interne ; et

insérer ledit sac de fluide (150) et ledit élément élastique (160) dans ledit élément d'enveloppe en forme de sac (140) présentant le jeu pour former ainsi ladite partie pliée au niveau de ladite enveloppe interne.

FIG.1

100

111

112

110

130

FIG.2

100

110

165

166

130

157

V          V

150

141
142    140

160

FIG.3

FIG.4

```
        ┌──────────────┐
        │    START     │
        └──────────────┘
               │
               ▼           ⌐S1
        ┌──────────────┐
        │INITIALIZATION│
        └──────────────┘
               │
               ▼           ⌐S2
        ┌──────────────┐
        │INCREASE CUFF │
        │  PRESSURE    │
        └──────────────┘
               │
               ▼           ⌐S3
        ┌──────────────┐
        │ REDUCE CUFF  │
        │  PRESSURE    │
        └──────────────┘
               │
               ▼           ⌐S4
        ┌──────────────┐
        │MEASURE BLOOD │
        │  PRESSURE    │
        └──────────────┘
               │
               ▼           ⌐S5
        ┌──────────────┐
        │DISPLAY BLOOD │
        │  PRESSURE    │
        └──────────────┘
               │
               ▼
        ┌──────────────┐
        │     END      │
        └──────────────┘
```

FIG.5

## FIG.6

## FIG.7

## FIG.8

S101
┌─────────────────────────────────┐
│ STACK CLOTH 41, SPACER 50, CLOTH 42, │
│ AND LOOP FASTENER 65            │
└─────────────────────────────────┘

S102
┌─────────────────────────────────┐
│ MELT AND BOND THUS STACKED CLOTH 41, │
│ CLOTH 42 AND LOOP FASTENER 65, AND CUT │
│ ALONG THE OUTER PERIPHERY OF THE │
│ BONDED PORTION                  │
└─────────────────────────────────┘

S103
┌─────────────────────────────────┐
│ TAKE OUT SPACER 50 VIA MOUTH 60 │
└─────────────────────────────────┘

S104
┌─────────────────────────────────┐
│ SEW ALONG THE BONDED PORTION OF │
│ CLOTH 41, CLOTH 42 AND LOOP     │
│ FASTENER 65                     │
└─────────────────────────────────┘

S105
┌─────────────────────────────────┐
│ INSERT AIR BAG 150 AND CURLED ELASTIC │
│ MEMBER 160 INTO BAG-SHAPED COVER │
│ MEMBER 140 VIA MOUTH 60, AND SEW UP │
│ THE MOUTH 60                    │
└─────────────────────────────────┘

## FIG.9

## FIG.10

FIG.11

S201

WITH CLOTH 42 BEING TUCKED, STACK
CLOTH 41, TUCKED CLOTH 42 AND LOOP
FASTENER 65

↓

S202

MELT AND BOND THUS STACKED CLOTH 41,
CLOTH 42 AND LOOP FASTENER 65, AND
CUT ALONG THE OUTER PERIPHERY OF
THE BONDED PORTION

↓

S203

SEW ALONG THE BONDED PORTION OF
CLOTH 41, CLOTH 42 AND LOOP
FASTENER 65

↓

S204

INSERT AIR BAG 150 AND CURLED ELASTIC
MEMBER 160 INTO BAG-SHAPED COVER
MEMBER 140 VIA MOUTH 60, AND SEW UP
THE MOUTH 60

FIG.12

FIG.13

FIG.14

FIG.15

FIG.16

FIG.17

FIG.18

## FIG.19

```
                                    ┌─ S301
┌─────────────────────────────────┐
│ FOLD AND IRON A PART OF RIM OF CUT│
│ CLOTH TO FORM INNER COVER 142     │
└─────────────────────────────────┘
                 │
                 ▼              ┌─ S302
┌─────────────────────────────────┐
│ WITH INNER COVER 142 BEING TUCKED,│
│ STACK OUTER COVER 141, TUCKED INNER│
│ COVER 142 AND LOOP FASTENER 165,  │
│ AND FOLD BACK FOLDED PORTION 141a │
│ OF OUTER COVER 141                │
└─────────────────────────────────┘
                 │
                 ▼              ┌─ S303
┌─────────────────────────────────┐
│ SEW OUTER PERIPHERY OF THUS STACKED│
│ OUTER COVER 141, INNER COVER 142 AND│
│ LOOP FASTENER 165                 │
└─────────────────────────────────┘
                 │
                 ▼              ┌─ S304
┌─────────────────────────────────┐
│ INSERT AIR BAG 150 AND CURLED ELASTIC│
│ MEMBER 160 INTO BAG-SHAPED COVER  │
│ MEMBER 140 VIA MOUTH 60, AND SEW UP│
│ THE MOUTH 60                      │
└─────────────────────────────────┘
```

## FIG.20

FIG.21

142

165

141a

141

142b

142b

FIG.22

141a

142

142c

XXIII

XXIII

60

142b

165

141

142b

FIG.23

FIG.24

FIG.25

FIG.26

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9117419 A
- JP 2002209858 A

- JP 2001231757 A
- JP 09117419 A